# EUROPEAN PATENT APPLICATION

(11) **EP 1 762 264 A1**
(43) Date of publication of application: **14.03.2007**
(21) Application number: 05255530.7
(22) Date of filing: 08.09.2005
(51) Int. Cl.: A61M 15/00, A61M 11/00

(54) **Ultrasonic nebulizer**

(71) Applicant: Quatek Co. Ltd., Nei Hu Taipei (TW)
(72) Inventor: Yu, Neng-Chih, Xi-Zhi Zhen, Taipei Xian (TW)
(74) Representative: Pratt, David Martin

(57) **Abstract**

The present invention discloses an improved nebulizer, comprising: an ultrasonic transducer, being connected to a nozzle plate having an island raised at the center portion thereof for enabling the nozzle plate to perform an action of ultrasonic vibration induced by the piezoelectric element placed inside the ultrasonic transducer; a chamber, for holding a liquid to be nebulized; and a fixing device, for fixing the ultrasonic transducer at a side of the chamber.

## Description

### FIELD OF THE INVENTION

The present invention relates to an improved nebulizer, and more particularly, to an improved ultrasonic nebulizer having a stool-like micro nozzle plate with an island outwardly bulging at the center portion thereof, which utilizes a ultrasonic transducer cooperating with the nozzle plate in order to generate an aerosol of small liquid droplets according to a Rayleigh Wave effect, that is, to enable a solution to be efficiently and rapidly nebulized.

### BACKGROUND OF THE INVENTION

A patient, who suffers from asthma or a chronic bronchial disease, may have to take an inhalation therapy, for example, to take a bronchiectatic spray when getting a trouble in breathing. Various nebulizers have been disclosed for nebulizing liquid medicine, enabling nebulized medicine to be easily inhaled by the patient, whereas the nebulization speed can be an important index for measuring the performance of an individual nebulizer

Conventional nebulizers can be divided into two categories: high-efficiency and reduced-efficiency. A high-efficiency nebulizer usually is a bulky device that can nebulizing a solution with a comparably better nebulization speed, but at the cost of more electricity consumed. On the other hand, a reduced-efficiency nebulizer usually is a small device that can nebulize a solution using less electricity, but at an inferior nebulization speed instead. Therefore, it is in great need to have a small nebulizer that can generate an aerosol of small liquid droplets with high nebulization speed while consumes less power.

### SUMMARY OF THE INVENTION

It is the primary object of the invention to provide an improved nebulizer, having a stool-like micro nozzle plate with an island bulging outwardly at the center portion thereof, which utilizes a ultrasonic transducer cooperating with the nozzle plate in order to generate an aerosol of small liquid droplets according to a Rayleigh Wave effect, that is, to enable a solution to be efficiently and rapidly nebulized.

To achieve the above object, the present invention provide an improved nebulizer, comprising:
an ultrasonic transducer, being connected to a nozzle plate having an island raised
at the center portion thereof for enabling the nozzle plate to perform an action of ultrasonic vibration induced by the piezoelectric element placed inside the ultrasonic-transducer;
a chamber, for holding a liquid to be nebulized; and
a fixing device, for fixing the ultrasonic transducer at a side of the chamber.

Other aspects and advantages of the present invention will become apparent from the following detailed description, taken in conjunction with the accompanying drawings, illustrating by way of example the principles of the present invention.

### BRIEF DESCRIPTION OF THE DRAWINGS

FIG. 1 is a front view of an improve nebulizer according to the present invention.
FIG. 2 is a side view of an improve nebulizer according to the present invention.

### DESCRIPTION OF THE PREFERRED EMBODIMENT

For your esteemed members of reviewing committee to further understand and recognize the fulfilled functions and structural characteristics of the invention, several preferable embodiments cooperating with detailed description are presented as the follows.

Generally, a ultrasonic transducer has an piezoelectric element made of lead zirconate titanate (PZT) piezoelectric ceramic, that as the piezoelectric element is driven to vibrate in a range of several micrometers by an alternating electrical field induced by coupling the ultrasonic transducer to an electric energy of a specific frequency, the ultrasonic transducer can use the Rayleigh wave effect to convert the alternating current to high-frequency acoustic energy and thus generate an aerosol of small liquid droplets.

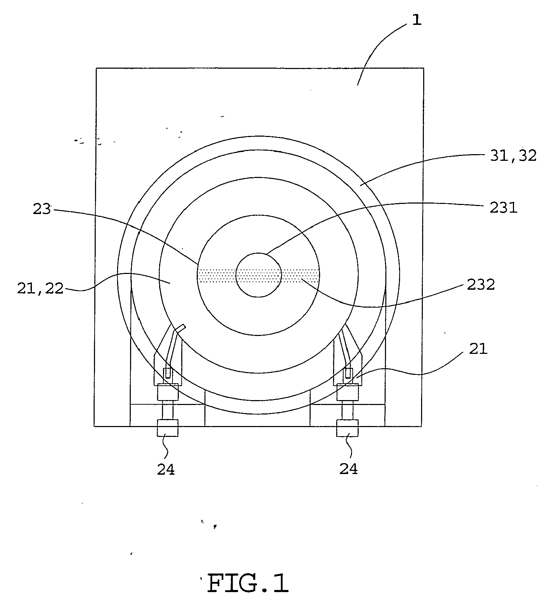

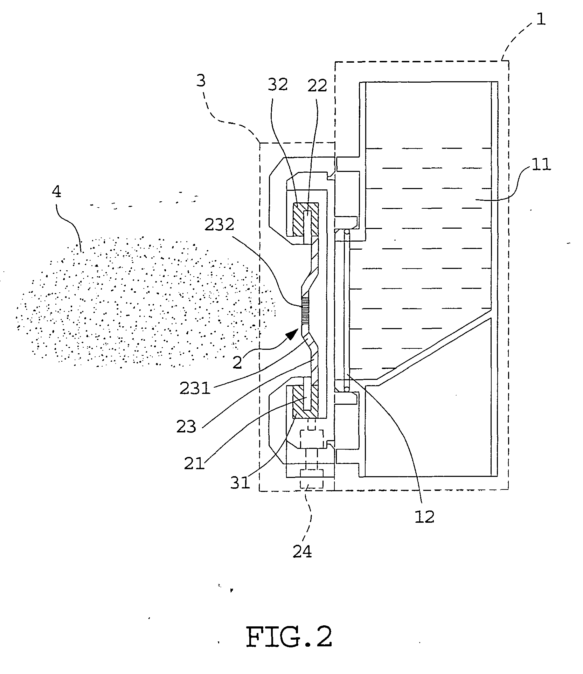

Please refer to FIG. 1 and FIG. 2, which are schematic views of an improve nebulizer according to the present invention. The improved nebulizer of the invention as seen in FIG. 1 and FIG. 2, comprises:
an ultrasonic transducer 2, being connected to a nozzle plate 23 having an island 231 raised at the center portion thereof for enabling the nozzle plate 23 to perform an action of ultrasonic vibration induced by a piezoelectric element 21 placed inside the ultrasonic transducer 2;
a chamber 1, for holding a liquid 11 to be nebulized, further having an O-ring 12 disposed at a position of the chamber 1 proximity to the nozzle plate 23 for preventing the liquid 11 from leaking; and
a fixing device 3, for fixing the ultrasonic transducer 2 at a side of the chamber 1, further having a glue 31 applied inside the fixing device 3 for pasting the nozzle plate 23 fixedly to the piezoelectric element 21;
wherein, the piezoelectric element 21 is driven by an alternating electrical field induced by coupling the ultrasonic transducer 2 to an electric energy of a specific frequency through two electrodes 24 while causes the ultrasonic transducer 2 to convert alternating current to high-frequency acoustic energy and thus generate an aerosol of small liquid droplets, and he island of the nozzle plate is a stool-like bulging structure formed by a means of prestress.

The operating principle of aerosol generation using the improved nebulizer of the invention is as following:
A voltage of specific frequency is coupled to the ultrasonic transducer 2, which causes the piezoelectric element 21 to deform or displace and thus to vibrate resonantly at the maximal so as to have an optimal nebulization efficiency, while the high-frequency acoustic energy converted by the ultrasonic transducer 2 is concentrated at the center portion of the nozzle plate 23
An island 231 raised at the center portion of the nozzle plate 23 is a stool-like bulging structure, which is formed by a means of prestress and capable of concentrating ultrasonic acoustic energy so as to increase the deformation/displacement of the piezoelectric element 21 and thus enable the ultrasonic transducer to vibrate resonantly at the maximal for raising nebulization efficiency thereof.
The nebulization efficiency of the improved nebulizer of the invention is increased while compared to the conventional nebulizer, since the area of the center portion of the stool-like nozzle plate 23 is larger than that of a conventional plane nozzle plate.

In operational, a liquid 11 to be nebulized is place in to the chamber and the weight of the liquid will enable the same to flux the island 231 of the nozzle plate 23. The same time that a voltage of specific frequency is applied over the two electrodes 24 enabling the ultrasonic transducer 2 to vibrate, so that the nozzle plate 23 connected to the ultrasonic transducer 2 is driven to vibrate axially, i.e. to vibrate in a direction perpendicular to the nozzle plate, and therefore the liquid 11 in the vicinity of the nozzle plate 23 is nebulized to generate an aerosol of small liquid droplets 4 by the Rayleigh wave effect. Moreover, the nebulization is stopped as the voltage of specific frequency is removed from the electrodes 24.

The improve nebulizer of the invention has advantages as following:
The small liquid droplets 4 generated by the nozzle plate 23 are homogeneous and are equal in size.
The nebulization efficiency of the improved nebulizer of the invention is increased, since the area of the center portion of the stool-like nozzle plate 23 is larger than that of a conventional plane nozzle plate and island 231 raised at the center portion of the nozzle plate 23 is capable of concentrating ultrasonic acoustic energy so as to increase the deformation/displacement of the piezoelectric element 21 and thus enable the ultrasonic transducer to vibrate resonantly at the maximal.

From the above description, it is noted that the present invention can provide an improved ultrasonic nebulizer having a stool-like micro nozzle plate with an island outwardly bulging at the center portion thereof, which utilizes a ultrasonic transducer cooperating with the nozzle plate in order to generate an aerosol of small liquid droplets according to a Rayleigh Wave effect, that is, to enable a solution to be efficiently and rapidly nebulized. With the characteristic of the stool-like nozzle plate having an island raised at the center portion thereof, it is clear that the nebulization efficiency of nebulizer of the present invention is better than that of a conventional nebulizer with plane nozzle plate.

While the preferred embodiment of the invention has been set forth for the purpose of disclosure, modifications of the disclosed embodiment of the invention as well as other embodiments thereof may occur to those skilled in the art. Accordingly, the appended claims are intended to cover all embodiments which do not depart from the spirit and scope of the invention.

## Claims

1. An improved nebulizer, comprising:
an ultrasonic transducer, being connected to a nozzle plate having an island raised at the center portion thereof for enabling the nozzle plate to perform an action of ultrasonic vibration induced by a piezoelectric element placed inside the ultrasonic transducer;
a chamber, for holding a liquid to be nebulized; and
a fixing device, for fixing the ultrasonic transducer at a side of the chamber.

2. The nebulizer of claim 1, wherein the piezoelectric element is driven by an alternating electrical field induced by coupling the ultrasonic transducer to an electric energy of a specific frequency while causes the ultrasonic transducer to convert alternating current to high-frequency acoustic energy and thus generate an aerosol of small liquid droplets.

3. The nebulizer of claim 1, wherein the island of the nozzle plate is a stool-like bulging structure formed by a means of prestress.

4. The nebulizer of claim 1, wherein the piezoelectric element is made of a lead zirconate titanate (PZT) piezoelectric ceramic.

5. The nebulizer of claim 1, wherein a glue is applied inside the fixing device for pasting the nozzle plate fixedly to the piezoelectric element.

6. The nebulizer of claim 1, wherein an O-ring is disposed at a position of the chamber proximity to the nozzle plate for preventing the liquid from leaking.

7. The nebulizer of claim 1, wherein the nebulizer is applied in a device selected from the group consisting of an inhaler, a mist maker, a skin care device, and an ionizer.

## Amended claims

### Amended claims in accordance with Rule 86(2) EPC.

**1.** A nebuliser comprising:
an ultrasonic transducer (2) connected to a nozzle plate (23) having an island (231) raised at the central portion thereof for enabling the nozzle plate to perform ultrasonic vibration induced by a piezoelectric element (21) placed inside the ultrasonic transducer;
a chamber (1) for holding a liquid (11) to be nebulised; and
a fixing device (3) for fixing the ultrasonic transducer at a side of the chamber, **characterised in that** the island of the nozzle plate is a stool-like bulging structure formed by a means of prestress.

**2.** A nebuliser as claimed in claim 1, wherein the piezoelectric element (21) is driven by an alternating electrical field induced by coupling the ultrasonic transducer (2) to an electric energy of a specific frequency, thereby causing the ultrasonic transducer to convert alternating current to high-frequency acoustic energy and generate an aerosol of small liquid droplets.

**3.** A nebuliser as claimed in claim 1 or claim 2, wherein the piezoelectric element (21) is made of a lead zirconate titanate (PZT) piezoelectric ceramic.

**4.** A nebuliser as claimed in any of claims 1 to 3, wherein glue is applied inside the fixing device (3) to fix the nozzle plate (23) to the piezoelectric element (21).

**5.** A nebuliser as claimed in any one of claims 1 to 4, further comprising an O-ring (12) disposed at a position in the chamber in proximity to the nozzle plate (23) for preventing the liquid from leaking.

**6.** A nebuliser as claimed in any one of claims 1 to 5, wherein the nebuliser forms part of a device selected from the group consisting of an inhaler, a mist maker, a skin care device, and an ioniser.
